# EUROPEAN PATENT APPLICATION

(11) **EP 4 516 805 A1**
(43) Date of publication of application: **05.03.2025**
(21) Application number: 23195060.1
(22) Date of filing: 04.09.2023
(51) Int. Cl.: C07K 14/005, C12N 15/86

(54) **VIRAL VECTOR FOR FIBROBLAST TRANSFECTION**

(71) Applicant: Medizinische Hochschule Hannover, 30625 Hannover (DE)
(72) Inventor: Thum, Thomas, 30659 Hannover (DE); Groß, Sonja, Hannover (DE); Bär, Christian, Hannover (DE); Büning, Hildegard, Hannover (DE)
(74) Representative: Taruttis, Stefan Georg

(57) **Abstract**

The invention provides a viral vector based on adeno-associated virus serotype 2 (AAV2,) which preferentially targets and transduces activated cardiac fibroblasts in vivo, the viral vector comprising a nucleic acid construct for an effector molecule as a transgene, the viral vector being characterized by comprising a capsid protein (CAP) which C-terminally and directly adjacent to amino acid No. 587 of the wild-type amino acid sequence of CAP contains an inserted amino acid section.

## Description

The present invention provides an AAV2-based viral vector suitable for targeting fibroblasts, especially activated fibroblasts, which are e.g. cardiac fibroblasts or myofibroblasts, and provides the viral vector for use in medical treatment of cardiac diseases associated with interstitial fibrosis, cardiac hypertrophy, or heart disease associated with or caused by pressure-overload.

The AAV2-based viral vector specifically targets activated fibroblasts, e.g. activated cardiac fibroblasts, or both activated cardiac fibroblasts and myofibroblasts, which activated fibroblasts have been found to be associated with pressure-overload of the heart. Accordingly, the viral vector is preferably provided for use in medical treatment of diseases associated with or caused by pressure-overload of the heart, by targeting activated cardiac fibroblasts. The viral vector of the invention, when provided with an expression cassette for a transgene results in permanent presence, preferably transduction and permanent expression of the transgene, in the targeted fibroblasts.

The viral vector has the advantage of preferentially targeting activated cardiac fibroblasts over non-activated or quiescent cardiac fibroblasts. The viral vector is provided for use in the genetic treatment of cardiac diseases which involve or are caused by cardiac fibroblasts, e.g. for use in the treatment subsequent to myocardial infarction, for use in the treatment of cardiac hypertrophy, or for use in the treatment of heart failure.

### State of the art

EP 2 850 096 B1 describes recombinant AAV-based vectors which in their VP3 protein have a mutation by replacing a specified position lysin (K490, K527, K549 or K556 of a specified wild-type CAP sequence) for glutamic acid and/or replacing K556 of this CAP by arginine.

### Object of the invention

It is an object of the invention to provide a viral vector based on AAV2 that preferentially targets and preferably transduces cardiac cells for expression of a transgene in cardiac cells, preferably in cardiac fibroblasts, more preferably for use in the treatment of a cardiac disease associated with cardiac fibrosis.

### Description of the invention

The invention achieves the object by providing a viral vector based on adeno-associated virus serotype 2 (AAV2, group AAV-A) which viral vector preferentially targets and transduces activated cardiac fibroblasts in vivo, the viral vector comprising a nucleic acid construct for an effector molecule as a transgene, the viral vector being characterized by comprising a capsid protein (CAP) which C-terminally and directly adjacent to amino acid No. 587 of the wild-type amino acid sequence of CAP contains an inserted amino acid section comprising or consisting of one of the amino acid sequences selected from SEQ ID NO: 4, SEQ ID NO: 14, SEQ ID NO: 5, SEQ ID NO: 10, and SEQ ID NO: 1. Preferably, the inserted amino acid section is arranged between amino acid No. 587 and amino acid No. 588 of the wild-type amino acid sequence of CAP (SEQ ID NO: 20).

The viral vector due to the CAP with one of these inserted amino acid section, arranged C-terminally to amino acid No. 587 of wild-type CAP, has the advantage of a high efficacy of infecting and transducing activated cardiac fibroblasts in vivo. Further, the viral vector of the invention can be produced efficiently in producer cells, e.g. for generating a high titer of the viral vector. The screening method uses a pressure-overload cardiac disease, induced by the TAC in the experimental animals. As a result, the three viral vectors that were identified as specific for cardiac fibroblasts, or in the case of two variants specific for cardiac fibroblasts and cardiomyocytes, are specific for activated cardiac fibroblasts as they occur in the cardiac disease state of pressure-overload.

A further important advantage of the viral vectors of the invention is that in a recipient they do not elicit antibodies that would affect, e.g. neutralize, a viral vector containing another one of the inserted amino acid sections. Accordingly, the viral vectors are suitable for use in medical treatment, e.g. for use in the treatment of a cardiac disease associated with pressure-overload, wherein subsequently two or three viral vectors of the invention are administered, wherein each of these viral vectors contains a different one of the inserted amino acid sections.

Further, the viral vectors of the invention have the advantage of preferentially targeting, e.g. transducing, cardiac fibroblasts over fibroblasts of other tissues. The viral vectors of the invention have the advantage of having a reduced tropism for other cell-types than cardiac fibroblasts, e.g. a lower tropism for liver tissue, and of expression of the nucleic acid sequence that is contained in the viral vector particle in cardiomyocytes, e.g. transgene expression at a level comparable to the level of expression of the same transgene from AAV9 and much higher than the level of expression of the same transgene from AAV2.

Cardiac fibroblasts are part of cardiac tissue, and the viral vector particle can be for medical use, e.g. for use in the treatment of cardiac diseases or cardiac defects, e.g. for administration to a human patient, wherein preferably or use in the treatment of a cardiac disease associated with cardiac fibrosis, e.g. for use in the treatment of cardiac fibrosis, or for use in the treatment of a cardiac disease associated with pressure-overload.

Optionally, the viral vector in addition to the inserted amino acid section comprises a linker sequence of 1 to 4 amino acids arranged between amino acid No. 587 of the wild-type amino acid sequence of CAP or amino acid No. 453 of the N-terminal section of wild-type CAP and the N-terminal amino acid of the inserted amino acid section. Therein, between the C-terminus of the inserted amino acid section and the remaining C-terminal section of CAP, no additional amino acid may be present so that the inserted amino acid section is directly adjacent to the amino acid sequence of the C-terminal portion of CAP, e.g. its amino acid No. 588. Alternatively, a linker sequence of 1 to 4 amino acids, e.g. of 1 to 3 amino acids, may be arranged between the C-terminus of the inserted amino acid section and the N-terminal amino acid of the remaining C-terminal section of CAP, e.g. its amino acid No. 588. Therein, the C-terminal section of CAP has the amino acid sequence of amino acids No. 588 to 735 of SEQ ID NO: 1.

The inserted amino acid section can be directly adjacent to the C-terminus of amino acid No. 587, respectively directly adjacent to the C-terminus of amino acid No. 588 of the wild-type amino acid sequence of CAP, or a linker sequence, e.g. of 1 to 5 amino acids, preferably of 3 amino acids, can be arranged between amino acid No. 587 of the wild-type amino acid sequence of CAP and the inserted amino acid section. The inserted amino acid can be directly adjacent to the N-terminus of amino acid No. 588 of the wild-type amino acid sequence of CAP, or a linker sequence, e.g. of 1 to 4 amino acids, preferably of 2 amino acids, can be arranged between the inserted amino acid section and the N-terminus of amino acid No. 588 of the wild-type amino acid sequence of CAP. An exemplary linker sequence for arrangement between amino acid No. 588 of the wild-type amino acid sequence of CAP and the N-terminus of the inserted amino acid section is ASA or AAA, an exemplary linker sequence for arrangement between amino acid No. 588 of the wild-type amino acid sequence of CAP and the C-terminus of the inserted amino acid section is AA. Preferably, the inserted amino acid section together with a linker sequence at its N-terminus and a linker sequence at its C-terminus consists of 16 to 7 amino acids, e.g. 14 to 7 amino acids, more preferably of 12 amino acids.

The linker section in each case can comprise or consist of at least one of the amino acids selected from Ala, Thr, Pro, Gly, Leu, and/or Ser, which amino acids can be different from one another or all the same in each linker section.

The viral vector particles contain a nucleic acid construct, e.g. a sense strand or an antisense strand of single-stranded DNA, comprising or consisting of an effector sequence between terminal ITR sequences of AAV2. The effector sequence can be an expression cassette encoding an effector molecule, e.g. encoding a functional non-coding RNA or a proteincoding RNA, which can be expressed from the vector construct in cardiac fibroblasts, which optionally are activated cardiac fibroblasts, and thereby exhibits a therapeutic beneficial function in cardiac fibroblasts and thus also for the whole heart.

Herein, transduction of cardiac fibroblasts is the introduction of nucleic acids by the viral vector particles into activated cardiac fibroblasts, which process can also be referred to as infection by the viral vector particles, especially for use of the viral vector particles in the treatment of activated cardiac fibroblasts. Generally, the viral vector particles can be for use in the treatment of activated cardiac fibroblasts, in vivo or in vitro, e.g. for the treatment of genetic defects of activated cardiac fibroblasts, especially for transduction of activated cardiac fibroblasts.

An exemplary effector molecule can be selected from any wild-type sequence, e.g. for use in complementing a defective gene in the recipient of the viral vector particle. Exemplary effector molecules are natural genes, including genes from any species, preferably human genes.

In a specific embodiment, empty viral particles are provided, which do not contain a nucleic acid molecule. These empty viral particles can e.g. be associated with a functional molecule for delivery of the functional molecule to heart tissue. The functional molecule can e.g. be a therapeutic agent or an indicator compound, e.g. a dye or a pharmaceutically acceptable diagnostic contrast agent, or a combination of at least two of these. Empty viral particles can be produced in HEK293 cells. The process comprised the steps of transfecting the respective helper plasmid (containing the *cap* gene, if applicable with an inserted amino acid section, and the *rep* gene of wild-type AAV2) and an adenoviral helper plasmid (containing adenoviral helper functions required for AAV vector production) in HEK293 cells for AAV empty capsid production (no vector genome plasmid containing ITRs flanking the expression cassette is used in this specific case), with subsequent purification of empty capsid particles by iodixanol gradient centrifugation.

Generally, the process for producing AAV viral vector particles according to the invention can be by delivery, e.g. by plasmid transfection with or without helper virus co-infection, of all required components for AAV vector production, e.g. from a vector genome containing the transgene expression cassette flanked by ITRs, AAV rep and AAV cap genes as well as other viral helper genes necessary for AAV particle production, e.g. from adenovirus. The process can be performed in a cultivated eukaryotic host cell, followed by cell lysis and removal of cellular components and plasmid DNA, e.g. by enzymatic digestion, filtration and/or centrifugation, and further purification, e.g. by gradient density centrifugation and/or chromatography of AAV viral vector particles. The AAV viral vector particles obtained by the process comprise the capsid protein (CAP) which C-terminally to amino acid No. 587 of the wild-type amino acid sequence of CAP contains an inserted amino acid section comprising one of the amino acid sequences selected from SEQ ID NO: 4, SEQ ID NO: 14, SEQ ID NO: 5, SEQ ID NO: 10, and SEQ NO: 1.

The viral vector particles can be formulated for injection, e.g. for direct injection into heart tissue, or for systemic injection, e.g. intravenous (i.v.) injection.

The transgene, also referred to as an effector molecule, encoded by a nucleic acid construct that is comprised in the viral vector can e.g. be the hepatocyte growth factor.

The invention is now described in greater detail by way of examples and with reference to the figures which show in
- Fig. 1 a schematic of a screening process identifying CAP variants that preferentially transduce activated cardiac fibroblasts,
- Fig. 2a a schematic of a secondary screening process identifying CAP variants by sequencing (NGS) of a barcode sublibrary,
- Fig. 2b results of analysis of cardiac fibroblasts (CF),
- Fig. 2c results of analysis of cardiomyocytes (CMC),
- Fig. 2d results of analysis of heart endothelial cells (EC),
- Fig. 2e results of analysis of liver,
- Fig. 2f results of analysis of spleen,
- Fig. 2g results of analysis of kidney,
- Fig. 2h results of analysis of lung,
- Fig. 2i results of analysis of muscle,
- Fig. 2j results of analysis of skin,
- Fig. 2k results of analysis of brain,
- Fig. 3 dot blot of high-temperature treated viral vectors,
- Fig. 4A results for transduction efficiency in HEK cells,
- Fig. 4B results of a heparin competition assay in transduction of HEK293 cells,
- Fig. 5A a schematic representation of the experimental set-up,
- Fig. 5B to 5E results for neutralizing antibodies against viral vectors,
- Fig. 6A a schematic of the experimental set-up,
- Fig. 6B to 6E in vivo results for transduction of different tissues by viral vectors.

### Example 1: Specificity of AAV2-based viral vector for cardiac fibroblasts in vivo

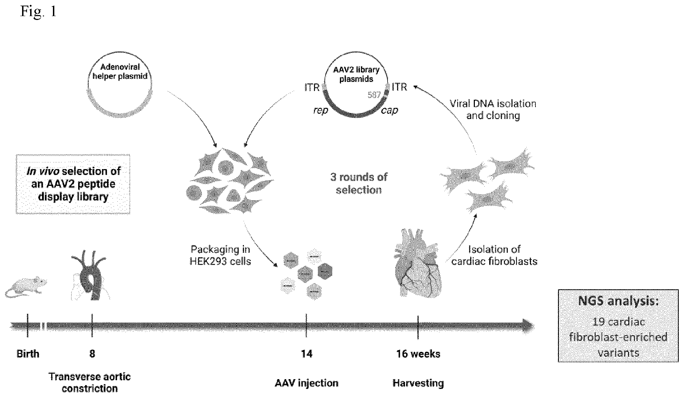

A murine model, a group of mice was used for pressure overload-induced hypertrophy for screening a large AAV2 capsid-modified library. Pressure overload was induced by performing transverse aortic constriction (TAC) banding, resulting in a hypertrophic heart with severe interstitial fibrosis after 6 weeks, when the AAV library was injected. Briefly, quiescent fibroblasts become activated in response to the mechanical stress induced by the TAC and differentiated into myofibroblasts, and later on into matrifibrocytes. Myofibroblasts are highly proliferative and express alpha smooth muscle actin (αSMA), a contractile fiber that allows wound closure and mechanical support. Formation of myofibroblasts can thus be considered as an acute response, whereas matrifibrocytes are responsible for the production of excessive amounts of extracellular matrix and specific matrix remodelling proteins (MMPs, matrix metalloproteinases) in the chronic phase. For screening, a library of AAV2 having different CAP, each containing an inserted amino acid section between amino acid No. 587 and No. 588 was used, which library contained individual nucleic acid sequences (so-called barcode library) for later identifying the individual inserted amino acid section. Identification of AAV2 variants was by next generation sequencing. The choice of the TAC animal model aimed to identify AAV capsid variants that predominantly target disease-associated fibroblasts and which preferably significantly less target quiescent fibroblasts. Since molecular patterns of activated and quiescent fibroblasts are quite similar, a complete specificity for activated cardiac fibroblast in combination with essentially absent targeting of quiescent fibroblasts was considered as probably not possible, but the screening identified AAV vectors that preferentially transduce activated cardiac fibroblasts. A schematic of the screening process is shown in Fig. 1.

The AAV2 library selected for this approach contains approximately four million different capsid variants. All of them contain a distinct insertion in the cap gene, C-terminally to amino acid position 587 (SEQ ID NO: 20, VP1 numbering) of the all three capsid proteins, VP1, 2 and 3. This position was selected, because it is the tip of a protrusion and contains key residues of the natural primary receptor binding motif, i.e. residues which interact with the cellular heparan sulfate proteoglycan (HSPG). By inserting seven random amino acids, the natural HSPG binding was affected and enabled respective capsid variants to differently interact with alternative receptors of target cells.

Disregarding the lack of an original cardiac tropism, AAV2 was chosen as library backbone, because it is a well understood serotype, and because it was found that screening led to viral vector variants that maintain their target cell specificity across species, especially in humans, despite being identified in a screening in mice.

In the producer cells, the following titers of viral vectors were produced:

| **aa sequence of inserted section** | **AAV variant #** | **Titer (particles /mL)** | |
|---|---|---|---|
| APSNATR | AAV-Var1 | 9.46 × 10⁹ | SEQ ID NO: 1 |
| RNLTQRD | AAV-Var 2 | 4.97 × 10⁸ | SEQ ID NO: 2 |
| TPGSLSR | AAV-Var 3 | 5.12 × 10⁸ | SEQ ID NO: 3 |
| AANQTRP | AAV-Var 4 | 1.52 × 10⁹ | SEQ ID NO: 4 |
| PGTINAP | AAV-Var 5 | 2.60 × 10⁷ | SEQ ID NO: 5 |
| VGRFINA | AAV-Var 6 | 2.50 × 10⁸ | SEQ ID NO: 6 |
| RGKQDGS | AAV-Var 7 | 3.88 × 10⁸ | SEQ ID NO: 7 |
| GLFAITP | AAV-Var 8 | 6.06 × 10⁶ | SEQ ID NO: 8 |
| VNQSPAR | AAV-Var 9 | 1.43 × 10⁷ | SEQ ID NO: 9 |
| GVAGVQA | AAV-Var 10 | 1.11 × 10¹⁰ | SEQ ID NO: 10 |
| STTGTVR | AAV-Var 11 | 3.46 × 10⁷ | SEQ ID NO: 11 |
| APGSLSR | AAV-Var 12 | 5.89 × 10⁴ | SEQ ID NO: 12 |
| DSHPPVR | AAV-Var 13 | 1.08 × 10³ | SEQ ID NO: 13 |
| VNTTRVG | AAV-Var 14 | 1.57 × 10¹⁰ | SEQ ID NO: 14 |
| PPAAGAR | AAV-Var 15 | 4.84 × 10² | SEQ ID NO: 15 |
| NRSQTPS | AAV-Var 16 | 1.89 × 10⁶ | SEQ ID NO: 16 |
| SSGVQVR | AAV-Var 17 | 1.96 × 10⁹ | SEQ ID NO: 17 |
| MPNTPTK | AAV-Var 18 | 1.30 × 10⁹ | SEQ ID NO: 18 |
| PTPSLSR | AAV-Var 19 | 1.08 × 10⁹ | SEQ ID NO: 19 |
| - | AAV2 wt | 6.91 × 10⁹ | SEQ ID NO: 20 |

After three rounds of selection in TAC mice, next-generation sequencing (NGS) revealed 19 variants which enriched in cardiac fibroblasts, showing high specificity for these cells in vivo. In order to use as few animals as possible, all variants simultaneously characterized with regard to their tropism and for being able to express in the target cell type. For this, a GFP-expressing barcode sublibrary was been generated, which under the control of a CMV promoter encoded eGFP as a marker protein, with an added individual 6-nucleotide section, which served as an individual label (barcode), arranged in 3' to the eGFP-encoding section, followed by a polyA site. With the exception of five variants which showed a low titer in production, all variants could be produced separately with good titers of viral particles. Then, the separate viral particles were pooled to produce a single sublibrary. This expression barcode sublibrary was again applied to screening in TAC mice (4 mice, 1.5×10⁹ vector genomes per mouse administered i.v.), followed by isolation of DNA and RNA from target and non-target cell types or organs. NGS analysis identified three variants highly enriched in the fibroblast fraction, namely containing an inserted amino acid section of SEQ ID NO: 1, SEQ ID NO: 4, and SEQ ID NO: 14, and two variants which in addition showed transduction of cardiomyocytes, namely SEQ ID NO: 5, and SEQ ID NO: 10, each inserted amino acid section arranged C-terminally to amino acid No. 587 of the wild-type CAP of SEQ ID NO: 20.

Variant 1 (inserted section SEQ ID NO: 1) showed the highest count of genomes in cardiac fibroblasts, corresponding to a high specificity for cardiac fibroblasts, followed by variant 14 (inserted section SEQ ID NO: 14) and variant 4 (inserted section SEQ ID NO: 4).

Astonishingly, variant 4 (Var4) by far achieved the best transduction efficiency (i.e. transgene mRNA expression) in cardiac fibroblasts. Further, variant 5 (Var5) and 10 (Var10) showed an improved transduction efficiency of cardiac fibroblasts compared to the wild-type AAV2, but additionally also transduced cardiomyocytes efficiently. Those five vector variants were selected for further characterization, firstly *in vitro.*

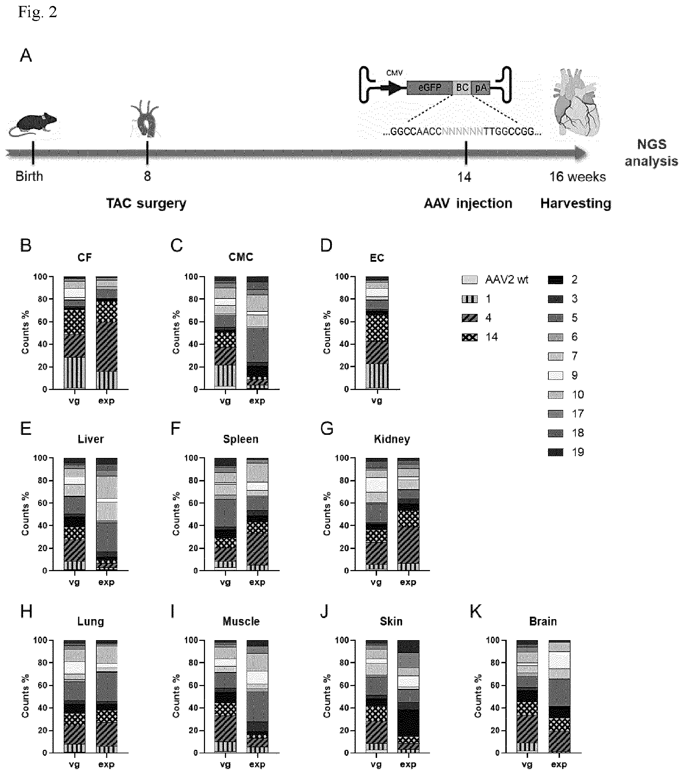

Fig. 2a schematically shows the in vivo selection of the GFP-barcode sublibrary. The barcode (BC) contains six nucleotides that are flanked by a linker sequence (SEQ ID NO: 21), arranged between eGFP and a polyA (pA) tail, TAC: transverse aortic constriction.

Fig. 2b to Fig. 2k show the results of analysis by NGS (vg = vector genomes) and by expression of the marker eGFP (exp = marker expression), showing transduction efficiency by viral vectors in different tissues. The results show that variant 1, variant 4 and variant 14 predominantly transduced cardiac fibroblasts in the TAC mice, and variant 5 and variant 10 predominantly transduced both cardiac fibroblasts and cardiomyocytes in the TAC mice.

### Example 2: In vitro characterization of viral vectors

The three AAV variants 1, 4 and 14 that were identified in the in vivo screening in TAC mice of example 1 were used to transduce neonatal mouse cardiac fibroblasts (NMCFs). The transduction efficiency was compared to the variants Var5 and Var10 that in TAC mice had been found to be enriched to a lower extent in cardiac fibroblasts but also in cardiomyocytes. The results for the NMCFs resembled the levels detected by the NGS analysis in the TAC mice. As an additional control, the ability of AAV6 to transduce mouse fibroblasts was examined, since AAV6 is known to infect hepatic fibroblasts and might thus be of interest in cardiac fibroblasts as well. The results indicated that AAV6 is not suitable for transducing NMCFs. Generally, in the examples, eGFP is used as a marker representing a transgene, e.g. being or encoding an effector molecule.

Next, the ability of the identified AAV variants to transduce human cardiac fibroblasts (HCFs) was assessed after 48 h (GOI: 1×10⁴). HCFs of different origin were analysed: Firstly, commercially available HCFs (Lot No. 1), and secondly, primary HCFs isolated from a biopsy of an end-stage heart failure patient. Microscopy showed that the wild-type AAV2 and variant 1 gave some eGFP-positive cells in the commercial HCFs, the end-stage heart failure HCFs seemed to be less susceptible. In both types of fibroblasts, AAV variant 4 and variant 14 resulted in a high number of eGFP-expressing cells already 48 h after transduction.

The same transduction pattern was observed in human embryonic lung fibroblasts (MRC-5 cells) after 48 h (GOI: 1×10⁴), indicating that the viral vectors of the invention can be applied in other organs, especially for use in the treatment of fibrosis-associated diseases, e.g. in the lung. When comparing the transduction efficiency with non-fibroblast cells within the heart such as neonatal mouse cardiomyocytes, it was found that variant 4 and variant 14 seemed to outperform the wild-type AAV2.

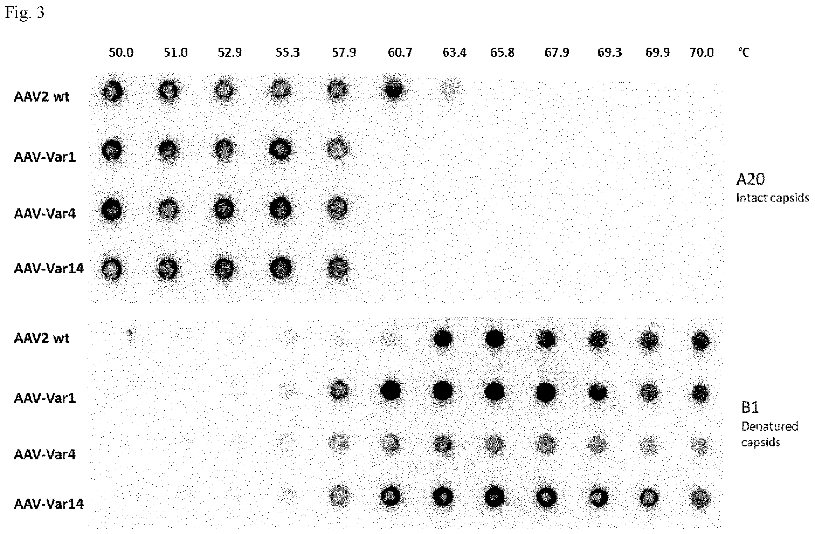

Characteristics of the fibroblast-tropic AAV variants of the invention were assessed for the three variants AAV-Var1, Var4 and Var14 as these in vitro in fibroblasts showed the highest GFP expression. A thermal stability assay based on antibody-mediated recognition of intact and denatured capsids was made. Thermal capsid stability assay was performed by incubating viral vector preparations AAV-Var1, AAV-Var4, AAVVar14, and of wild-type AAV2 (AAV wt) that were produced in producer cells for 15 min at the temperatures between 50.0 and 70.0 °C as indicated in Fig. 3. After the treatment at these elevated temperatures, samples were applied to a dot blot membrane and probed with A20 and B1 antibodies, respectively. A20 antibody recognizes intact AAV2 capsids (A20 Intact capsids in Fig. 3), while B 1 antibody recognizes the C termini of VP1, VP2 and VP3 which are located in the capsid interior in intact capsids. Thus, B1 antibody is used to detect denatured capsids (B1 Denatured capsids in Fig. 3). A slightly diminished capsid stability of all viral vectors of the invention compared to AAV2 wild-type capsids was observed. While all capsids were stable at 57.9 °C, only AAV2 wild type capsids resisted temperatures of 60.7 °C. AAV-Var1 appeared to have the least stable capsid. Recently, a decreased stability was correlated with an improved uncoating efficiency and thus, an increased transgene expression.

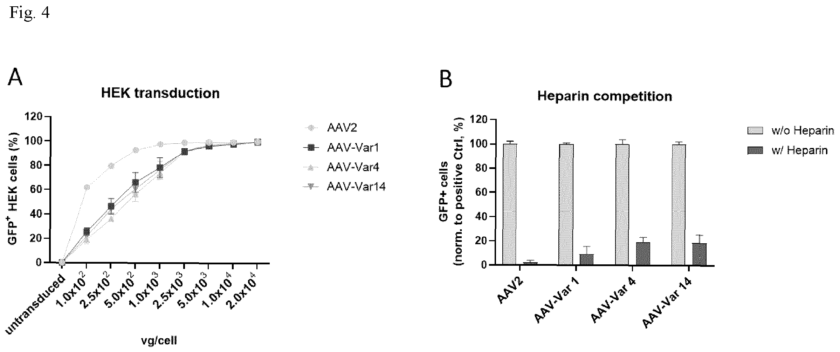

HEK293 cells are highly permissive for the wild-type AAV2. A change of tropism is therefore indicated by a reduced transduction of HEK293 cells. Increasing concentrations of viral vectors of the invention were used and the amount of eGFP-positive cells was quantified via flow cytometry after 48 h. Results are shown in Fig. 4A. The lowest concentration of AAV2 wild-type already resulted in 60% GFP-positive cells, while the AAV variants AAV-Var1, AAV-Var4 and AAV-Var14 achieved approximately 20% transduction. The increasing viral vector concentrations (viral genomes/cell = vg/cell) as indicated on the X-axis of Fig. 4 shows a reduced infectivity of the viral vectors of the invention in comparison to wild-type AAV2 (AAV2) for HEK293 cells.

The dependence of viral particles on HSPG for infection, HSPG being the natural primary receptor of AAV2, was examined by a heparin competition assay. Heparin is the soluble analogue of HSPG. Upon binding to the capsid, heparin impairs binding of capsids to HSPG. Fig. 4B shows results for the ability of AAV2 variants to infect HEK293 cells and their dependence on HSPG-mediated cell entry. Viral vectors AAV-Var1, AAV-Var4, AAV-Var14 and wild-type AAV2 (AAV2, Ctrl) were applied on HEK293 cells in the heparin competition assay in HEK293 cells, using a titer of 1×10² vector genome containing AAV particles per cell in the absence or presence of heparin (425 U/mL). GFP-positive cells were normalized to the respective wild-type AAV2 control (without heparin, w/o Heparin; with heparin, w/ Heparin). GFP-positive cells were determined via flow cytometry two days after transduction. n=2.

Transduction of cells by the wild-type AAV2 was almost completely abolished when heparin was present, but the proportion of GFP-positive cells was significantly higher when AAV variants according to the invention were used for infection of HEK293 cells.

A major hurdle for clinical use of viral vectors is the presence of neutralizing antibodies, either because of a previous immunization with wild-type AAVs or in the course of consecutive AAV-based gene therapies. For this reason, the potential of specific AAV wild-type antibodies to neutralize the viral vectors of the invention was assessed. Additionally, antibodies directed against either AAV-Var1, AAV-Var4 or AAV-Var14 were examined.

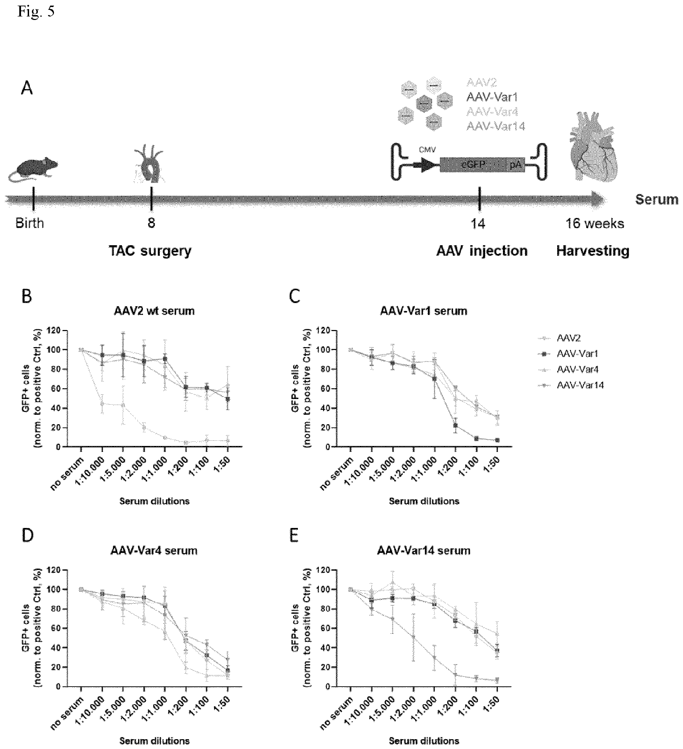

As a model for overload-pressure heart disease, mice were used at 6 weeks after TAC, injected with single preparations of viral vectors and after a period of another two weeks, which period was considered sufficient for allowing immune cells to produce neutralizing antibodies, mice were sacrificed and blood serum was prepared. Fig. 5A shows a schematic of the experiment.

This serum was pre-incubated (1 h at room temperature) in serial dilutions with the different AAV variants (vg/cell) before adding these serial dilutions containing viral vectors to HCFs in cell culture. At 48 h after addition of the serial dilutions containing viral vectors, GFP-positive cells were quantified via flow cytometry and the percentage was normalized to the positive control (AAV2 wild-type, Ctrl) without added serum (norm. to positive Ctrl, %). The results, shown in Fig. 5B for AAV2 wild-type with serum, Fig. 5C for AAV-Var1 with serum, Fig. 5D for AAV-Var4 with serum, Fig. 5E for AAV-Var14 with serum show that antibodies against the wild-type AAV2 reduced the amount of GFP-positive HCFs only slightly when transduced with the AAV variants of the invention, whereas wild-type AAV2 was effectively neutralized. With the highest serum concentration, still 60% of the maximum transduction efficiency was achieved for all three variants (Fig. 5B).

The AAV-Var1 and Var4 serum was most effective against the respective variant, but the other variants and wild-type AAV2 were also neutralized albeit a minor shift of the curve could be observed (Fig. 5C and Fig. 5D). Antibodies raised against AAV-Var14 were less specific for the other variants as indicated by the percentage of GFP-positive HCFs of approximately 40% with the lowest serum dilution (Fig. 5E). Since especially antibodies elicited by the wild-type AAV2 recognized the three AAV variants of the invention with a reduced specificity, the viral vectors of the invention are suitable for use in the treatment of patients, which are positive for AAV2 neutralizing antibodies.

### Example 3: In vivo characterization of viral vectors

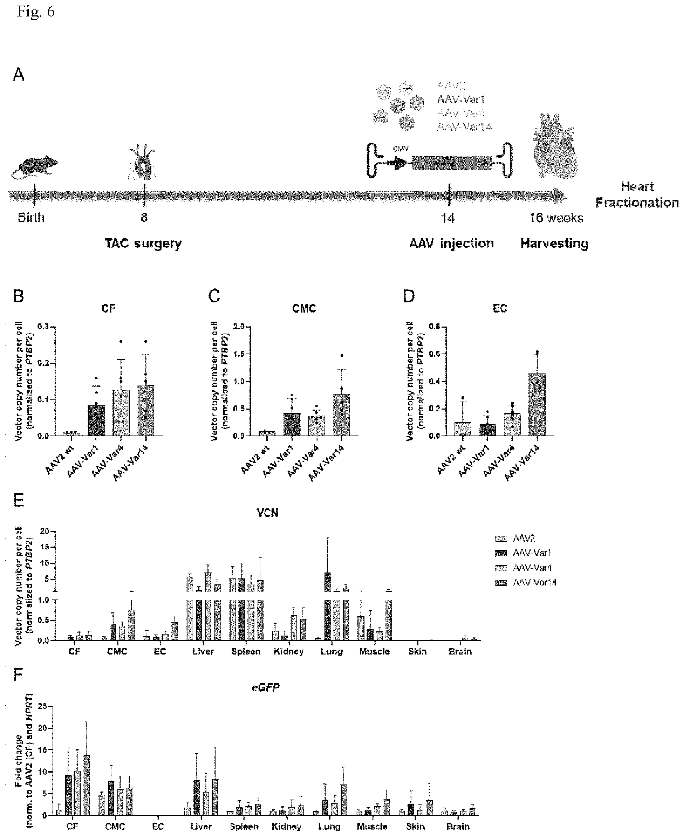

In order to test the distribution in vivo in a patient, the three AAV variants AAV-Var1, AAV-Var4 and AAV-Var14, and as a control wild-type AAV2 (AAV2 wt) were injected into mice (n= 3 to 6) at 6 weeks after TAC as a model for overload-pressure heart disease. Two weeks after the injection, organs were harvested, and a heart fractionation was conducted that enabled for a separate analysis of cardiac fibroblasts, cardiomyocytes and endothelial cells, as depicted in Fig. 6A. Vector genomes were determined via a RT-qPCR-based copy number analysis of the eGFP transgene, and these were normalized to the PTBP2 gene (vector copy numbers (VCN) per diploid cell). The improved infectivity of cardiac fibroblasts (CF) with the three AAV variants compared to the wild-type AAV2 was confirmed in vivo. Herein, AAV-Var4 and AAV-Var14 resulted in the highest copy numbers (Fig. 6B). Compared to AAV2, all three variants, furthermore, revealed an increased infectivity for cardiomyocytes (CMC), which was similar to the results found in in vitro experiments (Fig. 6C). In cardiac endothelial cells (EC), only AAV-Var14 demonstrated a greater infectivity (Fig. 6D) than wild-type AAV2.

Although copy numbers were lowest in cardiac fibroblasts compared to the other two cardiac cell types, the AAV variants of the invention demonstrated an improved infectivity of the cardiac fibroblasts, which is currently regarded as the most challenging cell type to transduce.

Apart from cardiac cell types, relevant off-target organs were analyzed (Fig. 6E). Most importantly, copy numbers in the liver and spleen were similar or even lower compared to infection by wild-type AAV2. In the kidney, both AAV-Var4 and AAV-Var14 showed an enhanced infectivity. Remarkably, the copy numbers in lung cells were similarly high as in the liver for all three variants, but wild-type AAV2 was almost below the detection threshold. Moreover, the skeletal muscle, being a target tissue of many AAV serotypes, was examined as well. In this tissue, only AAV-Var14 showed an increased copy number, whereas AAV-Var1 and AAV-Var4 were less specific than wild-type AAV2. The copy numbers in skin and brain tissue were very low, showing that these tissues were essentially not infected.

Since intracellular trafficking barriers might limit the transgene expression, qPCR analysis of the eGFP expression was additionally performed in these cell types and tissues. According to the VCN results, a stronger transgene expression was achieved in cardiac fibroblasts via the novel AAV variants of the invention when compared to the wild-type AAV. Most strikingly, the eGFP expression was highest in cardiac fibroblast cells transduced with the variants even compared to cardiomyocytes or the liver. Considering the higher copy numbers in both, an improved intracellular processing can at present be assumed in cardiac fibroblasts. This hypothesis is supported by the decreased thermal stability, which is pointing towards an increased uncoating efficiency of the AAV variants of the invention over the wild-type AAV2. These intracellular processes and barriers can differ substantially between different cell types and might explain the disparities between cell entry and transgene expression that were already observed in the barcode library sequencing results shown in Fig. 2.

## Claims

1. AAV2 viral vector comprising a nucleic acid construct for an effector molecule, **characterized by** comprising a capsid protein (CAP) which C-terminally to amino acid No. 587 of the wild-type amino acid sequence of CAP of SEQ ID NO: 20 contains an inserted amino acid section comprising one of the amino acid sequences of SEQ ID NO: 4, SEQ ID NO: 14, SEQ ID NO: 5, SEQ ID NO: 10, and SEQ ID NO: 1.

2. AAV2 viral vector particle according to claim 1, **characterized in that** a linker sequence of 1 to 4 amino acids is arranged between amino acid No. 587 of the N-terminal section of CAP and the N-terminal amino acid of the inserted amino acid section.

3. AAV2 viral vector particle according to one of the preceding claims, **characterized in that** a linker sequence of 1 to 3 amino acids is arranged between the C-terminus of the inserted amino acid section and the N-terminal amino acid of the remaining C-terminal portion of CAP.

4. AAV2 viral vector particle according to one of the preceding claims, **characterized in that** the remaining C-terminal portion of CAP are amino acids 588 to 735 of SEQ ID NO: 20.

5. AAV2 viral vector particle according to one of the preceding claims, **characterized by** the mutations R585A (amino acid 585 Arg to Ala) and R588A (amino acid 588 Arg to Ala).

6. AAV2 viral vector particle according to one of the preceding claims for use in the treatment of a disease or defect of cardiac fibroblasts, or in the treatment of a disease or defect of fibroblasts of the lung.

7. AAV2 viral vector particle for use in the treatment of a disease or defect of cardiomyocytes according to claim 6, **characterized in that** the vector particle contains a nucleic acid construct comprising an effector sequence.

8. AAV2 viral vector particle for use in the treatment of a disease or defect of cardiomyocytes according to claim 6 or 7, **characterized in that** the nucleic acid construct for an effector molecule is an expression cassette encoding an effector molecule.

9. AAV2 viral vector particle for use in the treatment of a disease or defect of cardiac fibroblasts according to one of claims 6 to 8, wherein the disease or defect is associated with activated fibroblasts in a heart with pressure-overload, cardiac hypertrophy, myocardial infarction, cardiac fibrosis, cardiotoxicity or cardiac failure.

10. AAV2 vector particle for use in the treatment of a disease or defect of cardiac fibroblasts according to one of claims 6 to 9, wherein the treatment is in vivo or ex vivo transduction of cardiomyocytes.

11. AAV2 viral vector particle for use in the treatment of a disease or defect of cardiac fibroblasts according to one of claims 6 to 10, wherein the person receiving the viral vector particle has antibody neutralizing wild-type AAV2 and/or has antibody neutralizing AAV9.

12. Process for producing AAV2 viral vector particles by delivery of components for AAV2 vector production in a cultivated eukaryotic cell, followed by cell lysis and removal of cellular components and plasmid DNA, and further purification of AAV2 viral vector particles, **characterized in that** the AAV2 viral vector particle comprises a capsid protein (CAP) which C-terminally to amino acid No. 587 of the wild-type amino acid sequence of CAP contains an inserted amino acid section comprising one of the amino acid sequences selected from SEQ ID NO: 1, SEQ ID NO: 4, SEQ ID NO: 14, SEQ ID NO: 5, and SEQ ID NO: 10.

13. Method of treatment of cardiac diseases, including cardiac defects, comprising administering an AAV2 vector particle according to one of claims 1 to 11 to a patient who is diagnosed to have a cardiac disease or cardiac defect.
